# EUROPÄISCHE PATENTANMELDUNG

(11) **EP 2 659 887 A1**
(43) Veröffentlichungstag der Anmeldung: **06.11.2013**
(21) Anmeldenummer: 12166649.9
(22) Anmeldetag: 03.05.2012
(51) Int. Cl.: A61K 31/166

(54) **Verwendung A-Ring-aromatisierter Acetyl-Minocycline und pharmazeutischer Zubereitungen daraus zur Therapie und Prophylaxe entzündlicher Erkrankungen, Autoimmunerkrankungen und Transplantatabstoßungen**

(71) Anmelder: Otto-von-Guericke-Universität Magdeburg, 39106 Magdeburg (DE)
(72) Erfinder: Kreutzmann, Peter, Dr., 39116 Magdeburg (DE); Lorenz, Peter, Dr., 39128 Magdeburg (DE); Reinhold, Dirk, Prof. Dr., 39108 Magdeburg (DE); Goihl, Alexander, 39112 Magdeburg (DE); Entz, Dominik, 39112 Magdeburg (DE); Franz, Claudia, 06862 Dessau-Roßlau (DE); Schraven, Burkhart, Prof. Dr., 39175 Biederitz (DE); Fischer, Klaus-Dieter, Prof. Dr., 39114 Magdeburg (DE)
(74) Vertreter: Schrell, Andreas

(57) **Zusammenfassung**

Die vorliegende Erfindung betrifft die Verwendung von A-Ringaromatisierten ein- oder mehrfachacetylierten Minocyclinen zur Behandlung und Prophylaxe von T-Zell-vermittelten Erkrankungen z. B. Autoimmunerkrankungen.

## Beschreibung

Die vorliegende Erfindung betrifft A-Ring-aromatisierte Minocyclinderivate, welche mindestens eine acetylierte Seitenkette tragen, insbesondere ein- oder mehrfachacetyliert sind, zur Verwendung in der Therapie von T-Zell-vermittelten Krankheiten und zur Suppression der Immunreaktion eines Patienten.

Es sind zahlreiche Krankheiten des menschlichen und tierischen Körpers bekannt, die mit der Immunreaktion des Körpers in Verbindung stehen. Dazu zählen einerseits Krankheiten des Immunsystems, im Rahmen derer körpereigene Stoffe fälschlicherweise als fremd erkannt werden und die als Autoimmunerkrankungen bezeichnet werden. Darüber hinaus können aber auch an sich harmlose Stoffe aus der Umwelt vom Immunsystem als gefährlich erkannt werden und so zu überschießenden Reaktionen führen, die im Allgemeinen als Allergie bezeichnet werden. Die Komplexität der Krankheitsbilder und des Immunsystems erschweren die Identifizierung und Entwicklung therapeutischer Verfahren ungemein. Obgleich mittlerweile eine Reihe an immunsuppressiven Pharmazeutika bekannt ist, z. B. Glucocortikoide, Zytostatika, Calcineurin-Inhibitoren, Proliferationshemmer oder Antikörper, stellt die mangelnde Selektivität und Effektivität dieser Pharmazeutika nach wie vor eine Herausforderung für die forschende Pharmazeutik dar.

Minocyclin ist ein semisynthetisches Breitband-Antibiotikum aus der Gruppe der Tetracycline und wird aus diesem Grund seit vielen Jahren in der klinischen Praxis zur Behandlung infektiöser Erkrankungen z.B. der Atemwege, des Urogenital- und Gastrointestinaltraktes, bei verschiedenen Hauterkrankungen, wie Akne vulgaris, Rosacea, sowie bei Trachom, Clamydien-Conjunctivitis, Borreliose etc. eingesetzt. Neben dieser antibiotischen Wirkung weist Minocyclin offenbar auch schützende Effekte in Tiermodellen verschiedener neurodegenerativen Erkrankungen auf [1]. Es konnte u. a. gezeigt werden, dass Minocyclin das Absterben von Neuronen infolge cerebraler Ischämie verhindern kann und inflammatorische Antworten/Reaktionen inhibiert.

Das breite Wirkungsspektrum von Minocyclin auf neuroinflammatorische Prozesse wird unter anderem mit der Inhibierung verschiedener pro-apoptotischer Faktoren erklärt, wie z.B. von Matrixmetalloproteasen [2], der induzierbaren Stickoxidsynthase (iNOS), der Freisetzung von mitochondrialem Cytochrom c und verschiedener Caspasen, sowie der Mikroglia-Aktivierung. Aber auch eine Einflussnahme von Minocyclin auf freie Radikale, reaktive Sauerstoff- und Stickstoffspezies, die allgemein als Auslöser pro-inflammatorischer und pro-apoptotischer Prozesse gelten, wird diskutiert [3, 4].

Aufgrund der gefundenen biologischen und pharmakologischen Wirkungen ist die Verwendung von Minocyclin zur Behandlung neurodegenerativer und inflammatorischer Erkrankungen interessant und die Untersuchung von Minocyclin als Leitstruktur stellt einen attraktiven Ansatz bei der Suche nach neuroprotektiven und immunmodulierenden Wirkstoffen dar.

Allerdings sind auch nachteilige Wirkungen von Minocyclin publiziert [5, 6]. So wird die schädliche Auswirkung von Minocyclin auf Mitochondrien bei Lebertransplantationen [7] und die lebertoxische Wirkung [8] diskutiert. Weiterhin kann es nach Applikation von Minocyclin zu Medikamenten-induzierten Autoimmunprozessen (Minocyclininduzierter Lupus) kommen [9-11]. All diese unerwünschten Nebenwirkungen schränken die Einsatzmöglichkeiten von Minocyclin stark ein.

In dem Versuch, die Einsatzmöglichkeiten von Minocyclin zu erweitern, wurden daher auch Derivate des Minocyclins entwickelt. Zu diesen Derivaten zählen auch A-Ring-aromatisierte Minocycline. A-Ring-aromatisierte Minocyclinderivate sind formal betrachtet Dehydroderivate des Minocyclins.

Aus der WO 2009/012741 A1 sind Verfahren zur Synthese von A-Ring-aromatisierten Acetylminocyclinen, insbesondere Penta- und Tetraacetylcycline, bekannt. Diesen A-Ring-aromatisierten Acetylminocyclinen wird eine Wirksamkeit für die Behandlung von durch oxidativen Stress und/oder mitochondriale Schädigungen vermittelten neurodegenerativen Erkrankungen zugeschrieben. Die Therapie von unmittelbar mit einer Immunreaktion, insbesondere einer zellulären Immunreaktion, in Zusammenhang stehenden Krankheiten wird nicht untersucht.

Den Anwendungsmöglichkeiten von Minocyclin und dessen Derivaten sind daher Grenzen gesetzt. Nach wie vor besteht ein dringender Bedarf mit einer Immunreaktion in Verbindung stehende Krankheiten, insbesondere Autoimmunerkrankungen und Allergien, zu behandeln.

Der vorliegenden Erfindung liegt daher das technische Problem zugrunde, die vorstehenden Nachteile überwindende, insbesondere verbesserte Therapiemöglichkeiten für mit der Immunreaktion eines Patienten in Zusammenhang stehende Krankheiten, insbesondere von T-Zell-vermittelten Krankheiten, bereitzustellen, insbesondere solche, die eine hohe Spezifität für ein bestimmtes Krankheitsbild und wenig bis keine Nebenwirkungen aufweisen.

Die vorliegende Erfindung löst das ihr zugrundeliegende technische Problem durch die Bereitstellung der Lehren gemäß der unabhängigen Ansprüche.

Insbesondere löst die vorliegende Erfindung das ihr zugrunde liegende technische Problem durch die Bereitstellung eines A-Ring-aromatisierten Acetylminocyclins der Formel I 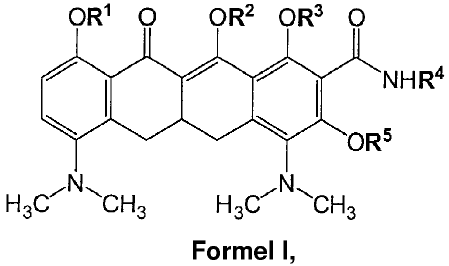 wobei R¹ bis R⁵ unabhängig voneinander = Acetyl und/oder H und wenigstens ein Rest R von R¹ bis R⁵ Acetyl ist, zur Verwendung in einem therapeutischen Verfahren zur Therapie von T-Zell-vermittelten Krankheiten.

Die vorliegende Erfindung stellt daher A-Ring-aromatisierte Acetylminocycline der Formel I bereit, das heißt A-Ring-aromatisierte ein- oder mehrfach acetylierte Minocycline. Diese A-Ring-aromatisierten Acetylminocycline weisen an einer, mehreren oder allen Positionen R¹, R², R³, R⁴ oder R⁵ einen Acetylrest, vorzugsweise insgesamt vier oder fünf Acetylreste (CH₃CO-), auf. Die erfindungsgemäßen A-Ring-aromatisierten Acetylminocycline weisen demgemäß mindestens einen Acetylrest in Form von R¹, R², R³, R⁴ oder R⁵ auf.

Im Zusammenhang mit der vorliegenden Erfindung werden unter A-Ring-aromatisierten Acetylminocyclinen der Formel 1, insbesondere der Formeln II oder III, einerseits die ein- oder mehrfach acetylierten A-Ring-aromatisierten Minocycline der Formeln I, insbesondere der Formeln II oder III, verstanden, andererseits aber auch deren funktionelle Äquivalente, insbesondere auch biologisch aktive und zirkulierende Fragmente, Analoga und/oder Derivate, solange deren biologische Aktivität mit der der in den Formeln I, II und III erfassten Verbindungen übereinstimmt. Derartige Derivate können insbesondere auch Salze oder Ionen, insbesondere Anionen, der A-Ring-aromatisierten Acetylminocycline der Formel I, insbesondere der Formeln II und III, sein. In einer besonders bevorzugten Ausführungsform werden unter den A-Ring-aromatisierten Acetylminocyclinen der Formeln I, II oder III exakt die Verbindungen verstanden, die von diesen Formeln dargestellt werden unter Ausschluss von funktionellen Äquivalenten, insbesondere auch biologisch aktiven und zirkulierenden Fragmenten, Analoga und/oder Derivaten.

Im Zusammenhang mit der vorliegenden Erfindung wird unter einem therapeutischen Verfahren ein Verfahren zur Vorbeugung oder Behandlung von Krankheiten verstanden, das insbesondere der Heilung, der Linderung von Symptomen, der Beseitigung von Symptomen oder Vorbeugung der Entwicklung von Symptomen und vorzugsweise der Wiederherstellung der körperlichen Unversehrtheit dient. In einer bevorzugten Ausführungsform der vorliegenden Erfindung wird unter einem therapeutischen Verfahren also auch ein Verfahren verstanden, das einerseits der Behandlung einer Krankheit dient andererseits aber auch prophylaktisch, das heißt vorbeugend, wirkt und daher dem Entstehen einer Krankheit vorbeugt. In einer anderen bevorzugten Ausführungsform wird unter einem therapeutischen Verfahren ein Verfahren zur Behandlung von Krankheiten verstanden, das demgemäß allein der Heilung, Beseitigung oder Linderung von Symptomen und vorzugsweise der Wiederherstellung der körperlichen Unversehrtheit dient. Die erfindungsgemäß eingesetzten A-Ring-aromatisierten Acetylminocycline werden am oder im menschlichen oder tierischen Körper eines Patienten eingesetzt, der der erfindungsgemäßen Therapie bedarf, insbesondere in einer pharmakologisch wirksamen Dosis.

In einer besonders bevorzugten Ausführungsform der vorliegenden Erfindung ist die T-Zell-vermittelte Krankheit eine T-Zell-vermittelte Allergie, insbesondere allergische Rhinitis, allergisches Asthma bronchiale, eine Kontaktallergie, insbesondere ein Kontaktekzem oder eine Arznei- und/oder Nahrungsmittelallergie.

In einer besonders bevorzugten Ausführungsform der vorliegenden Erfindung ist die T-Zell-vermittelte Krankheit eine Autoimmunerkrankung, insbesondere Multiple Sklerose, Rheumatoide Arthritis, Lupus erythematodes, insulin-abhängiger Diabetes mellitus (IDDM), Morbus Crohn, Colitis ulcerosa, Psoriasis, Glomerulonephritis, Vaskulitis, Uveitis, autoimmune Schilddrüsenerkrankungen, Zöliakie oder Morbus Addision.

In einer besonders bevorzugten Ausführungsform der vorliegenden Erfindung ist die T-Zell-vermittelte Krankheit eine bei einer Transplantation, insbesondere Organtransplantation, auftretende akute oder chronische Abstoßungsreaktion.

Die Erfindung betrifft auch A-Ring-aromatisiertes Acetylminocyclin der Formel I 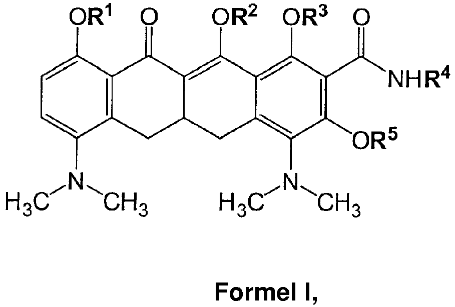 wobei R¹ bis R⁵ unabhängig voneinander Acetyl und/oder H und wenigstens ein Rest R von R¹ bis R⁵ Acetyl ist, zur Verwendung in einem therapeutischen Verfahren zur Suppression der Immunreaktion eines Patienten, insbesondere der zellulären Immunreaktion.

Die vorliegenden A-Ring-aromatisierten Acetylminocycline weisen eine hohe Suppressionsspezifität auf, insbesondere supprimieren sie vorzugsweise allein die zelluläre Immunreaktion, insbesondere die T-Zell-Vermehrung und/oder T-Zell-Zytokin-Bildung. Andere Komponenten des Immunsystems werden vorteilhafter Weise nicht supprimiert oder modifiziert.

Die vorliegenden A-Ring-aromatisierten Acetylminocycline eignen sich daher als Immunsuppressivum, insbesondere der zellulären Immunreaktion.

In einer besonders bevorzugten Ausführungsform der vorliegenden Erfindung ist die Suppression der Immunreaktion eine Reduktion der Proliferation, hier auch als Zellteilung bezeichnet, von T-Lymphozyten und/oder eine Reduktion der T-Zell-Zytokin-Bildung.

In bevorzugter Ausführungsform ist die Reduktion der Proliferation eine Reduktion oder Hemmung der DNS-Synthese in T-Lymphozyten.

In bevorzugter Ausführungsform ist die Reduktion der T-Zell-Zytokin-Bildung eine Reduktion der Bildung von IL-2, IFN-y und/oder IL-17.

In einer besonders bevorzugten Ausführungsform sieht die Erfindung daher vor, die A-Ring-aromatisierten Acetylminocycline der Formel I zur Suppression einer Immunreaktion eines Patienten zu verwenden. In besonders bevorzugter Ausführungsform wird die Suppression der Immunreaktion bei der Therapie von T-Zell-vermittelten Krankheiten verwendet. In einer besonders bevorzugten Ausführungsform werden die A-Ring-aromatisierten Acetylminocycline der Formel I daher zur Suppression der Immunreaktion eines Patienten bei der Therapie von T-Zell-vermittelten Krankheiten, insbesondere T-Zell-vermittelten Allergien, T-Zell-vermittelten Autoimmunerkrankungen oder zur Therapie von bei einer Transplantation auftretenden Abstoßungsreaktionen verwendet.

In einer besonders bevorzugten Ausführungsform der vorliegenden Erfindung ist das A-Ring-aromatisierte Acetylminocyclin gemäß der Formel I Pentaacetylcyclin gemäß der Formel II

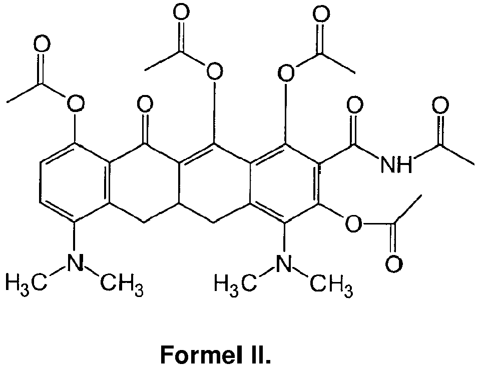

In einer besonders bevorzugten Ausführungsform der vorliegenden Erfindung ist das A-Ring-aromatisierte Acetylminocyclin gemäß der Formel I Tetraacetylcyclin gemäß der Formel III

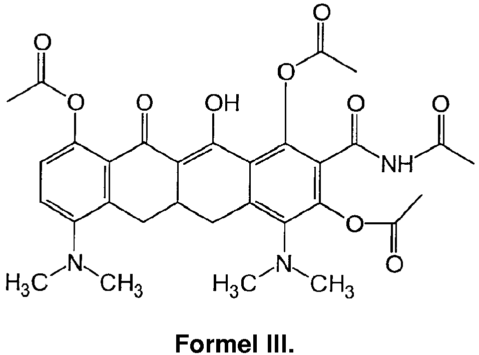

Die Herstellung der erfindungsgemäß eingesetzten A-Ring-aromatisierten Acetylminocycline wird in der WO 2009/012741 A1, insbesondere den Beispielen 1 und 2, beschrieben, die hinsichtlich der Herstellung der vorliegenden A-Ring-aromatisierten Acetylminocycline vollständig in den Offenbarungsgehalt der vorliegenden Lehre mit einbezogen ist.

Die vorliegende Erfindung betrifft auch eine pharmazeutische Zusammensetzung zur Verwendung in einem therapeutischen Verfahren zur Suppression der Immunreaktion in einem Patienten, umfassend wenigstens ein A-Ring-aromatisiertes Acetylminocyclin der Formel I, insbesondere Formel II oder III, wobei R¹ bis R⁵ unabhängig voneinander Acetyl oder H und wenigstens ein Rest R von R¹ bis R⁵ Acetyl ist, und einen pharmazeutisch verträglichen Träger.

Die vorliegende Erfindung betrifft auch eine pharmazeutische Zusammensetzung zur Verwendung in einem therapeutischen Verfahren zur Therapie von T-Zell-vermittelten Krankheiten, umfassend wenigstens ein A-Ring-aromatisiertes Acetylminocyclin der Formel I, insbesondere Formel II oder III, wobei R¹ bis R⁵ unabhängig voneinander Acetyl oder H und wobei wenigstens ein Rest R von R¹ bis R⁵ Acetyl ist, und einen pharmazeutisch verträglichen Träger.

Bevorzugt ist die pharmazeutische Zusammensetzung gemäß der vorliegenden Erfindung für die systemische Anwendung, insbesondere zur oralen, transdermalen, percutanen, intravenösen, subcutanen, intracutanen, intramuskulären, rektalen, vaginalen oder sublingualen Applikation, geeignet, insbesondere zusammen mit an sich bekannten Träger-, Hilfs- und/oder Zusatzstoffen.

Bevorzugt ist die pharmazeutische Zubereitung gemäß der vorliegenden Erfindung für die topische Anwendung geeignet, z.B. in Form von Cremes, Salben, Pasten, Gelen, Lösungen, Sprays, Lipo- bzw. Nanosomen, "pegylierten" Formulierungen, degradierbaren Depot-Matrices, Schüttelmixturen oder Hydrokolloidverbänden, insbesondere mit anderen dermatologischen Grundlagen oder Vehikeln, einschließlich instillativer Applikation.

In besonders bevorzugter Ausführungsform enthalten die vorliegenden pharmazeutischen Zusammensetzungen den Wirkstoff gemäß der vorliegenden Erfindung als wirksamen Bestandteil von galenischen Formen und in Verbindung mit einem geeigneten Carrier zur oralen, intravenösen, intramuskulären, intracutanen, intrathekalen Anwendung oder als Aerosol zur transpulmonalen Applikation.

Die vorliegende Erfindung betrifft auch Verfahren zur Therapie von T-Zell-vermittelten Krankheiten und/oder zur Suppression der Immunreaktion eines menschlichen oder tierischen Körpers, insbesondere eines Patienten, wobei dem menschlichen oder tierischen Körper ein A-Ring-aromatisiertes Acetylminocyclin der Formel I, insbesondere der Formeln II und/oder III, in einer Menge geeignet zur Behandlung der Krankheiten verabreicht wird und dabei eine Vorbeugung oder Behandlung der T-Zell-vermittelten Krankheit und/oder eine Immunsuppression erreicht wird.

Die vorliegende Erfindung betrifft auch die Verwendung von A-Ring-aromatisierten Acetylminocyclinen der Formel I, insbesondere der Formeln II und/oder III, zur Herstellung einer pharmazeutischen Zusammensetzung zur Behandlung von T-Zell-vermittelten Krankheiten und/oder zur Suppression der Immunreaktion eines menschlichen oder tierischen Körpers, insbesondere Patienten.

Der Erfindung liegt der Befund zugrunde, dass Pentaacetylcyclin, im Gegensatz zur Vergleichssubstanz Minocyclin, in *in vitro-*Experimenten bereits in niedriger Dosierung, z. B. <5,0 µM, die Proliferation, insbesondere DNS-Synthese, von Immunzellen hemmt (vergleiche auch Beispiel 1 und Beispiel 3) und gleichzeitig auch die Freisetzung inflammatorischer Zytokine deutlich vermindert (vergleiche auch Beispiel 2 und Beispiel 4).

Auch in einem *in* vivo-Mausmodell der Multiplen Sklerose (MS), der Experimentellen Autoimmunen Enzephalomyelitis (EAE), konnte die immunsuppressive Wirkung von Pentaacetylminocyclin eindeutig nachgewiesen werden. Der Wirkstoff Pentaacetylminocyclin zeigte sowohl bei therapeutischer intraperitonealer Applikation als auch überraschenderweise bei oraler Applikation einen signifikanten Einfluss auf die klinischen Symptome und den Schweregrad der EAE in der akuten Phase der Erkrankung (vergleiche auch Beispiel 5). Interessanterweise konnte beobachtet werden, dass Pentaacetylminocyclin im Vergleich zu Minocyclin etwa 4 bis 6 Tage früher eine signifikante Wirkung auf den Schweregrad der Erkrankung ausübte. Diese schnellere Verbesserung der klinischen Symptome stellt einen Vorteil der vorliegenden A-Ring-aromatisierten Acetylminocycline, insbesondere des Pentaacetylcyclins, dar. Untersuchungen der entzündlichen Zellinfiltrate im Rückenmark nach EAE (vergleiche auch Beispiel 6) bestätigen die der vorliegenden Erfindung zugrunde liegenden *in* vivo-Daten. All diese Befunde sind überraschend und zeigen die herausragenden Wirkeigenschaften der vorliegenden Wirkstoffe, insbesondere der beispielhaft getesteten Substanz. Die im Vergleich zu Minocyclin stärkere antiinflammatorische/immunmodulatorische Wirkung von Pentaacetylcyclin *in vitro* und *in vivo* belegt das hohe therapeutische Potential der A-Ring-aromatisierten Acetylminocycline.

Da für A-Ring-aromatisierte Acetylminocycline bisher keine antibiotische Wirkung nachgewiesen werden konnte, besteht bei der therapeutischen und/oder prophylaktischen Verwendung von Pentaacetylminocyclin keine Gefahr einer Ausbildung von Antibiotikaresistenzen, was ein weiterer Vorteil gegenüber dem Antibiotikum Minocyclin ist.

Weitere bevorzugte Ausführungsformen ergeben sich aus den Unteransprüchen.

Die vorliegende Erfindung wird anhand der folgenden Beispiele und der dazugehörigen Figuren näher beschrieben.

Die Figuren zeigen:
Figuren 1 A und B zeigen den Einfluss von Minocyclin und Pentaacetylcyclin auf die DNS-Synthese PWM-stimulierter (A) bzw. anti-CD3-stimulierter (B) humaner T-Lymphozyten. Dargestellt sind die Mittelwerte und Standardfehler der Mittelwerte von vier unabhängigen Experimenten.
   * kennzeichnet signifikante Verminderung zu Kontrollkulturen ohne Wirkstoff (p < 0,05).
Figuren 2A und B zeigen den Einfluss von Minocyclin und Pentaacetylcyclin auf die Vitalität PWM-stimulierter (A) bzw. anti-CD3-stimulierter (B) humaner T-Lymphozyten. Dargestellt sind die Mittelwerte und Standardfehler der Mittelwerte von drei unabhängigen Experimenten.
Figuren 3A, B und C zeigen die Produktion der Zytokine IL-2 (A), IFN-y (B) und IL-17 (C) nach Stimulation humaner T-Lymphozyten mit PWM und 48-stündiger Inkubation in An- und Abwesenheit verschiedener Konzentrationen Minocyclin und Pentaacetylcyclin. Dargestellt sind die Mittelwerte und Standardfehler der Mittelwerte von vier Experimenten.
   * kennzeichnet signifikante Verminderung zu Kontrollkulturen ohne Wirkstoff (p < 0,05).
Figuren 4A und B zeigen den Einfluss von Minocyclin und Pentaacetylcyclin auf die DNS-Synthese PWM-stimulierter (A) bzw. anti-CD3-stimulierter (B) SJL/J-Maus-Splenozyten. Dargestellt sind die Mittelwerte und Standardfehler der Mittelwerte von mindestens drei unabhängigen Experimenten.
   * kennzeichnet signifikante Verminderung zu Kontrollkulturen ohne Wirkstoff (p < 0,05).
Figuren 5A und B zeigen den Einfluss von Minocyclin und Pentaacetylcyclin auf die Vitalität PWM-stimulierter (A) bzw. anti-CD3-stimulierter (B) Maus-Splenozyten. Dargestellt sind die Mittelwerte und Standardfehler der Mittelwerte von drei unabhängigen Experimenten.
Figuren 6A und B zeigen Freisetzung von IFN-y (A) bzw. IL-17 (B) nach 48 Stunden aus mit PWM-stimulierten Splenozyten von SJL/J-Mäusen und Zugabe verschiedener Konzentrationen Minocyclin oder Pentaacetylcyclin. Dargestellt sind die Mittelwerte und Standardfehler der Mittelwerte von vier Experimenten.
   * kennzeichnet signifikante Verminderung zu Kontrollkulturen ohne Wirkstoff (p < 0,05).
Figuren 7A und B zeigen die Krankheitsverläufe der EAE (EAE-Score) in weiblichen SJL/J-Mäusen, welche nach Ausbildung der Erkrankung intraperitoneal (A, von Tag 12 bis Tag 20) oder oral (B, von Tag 14 bis Tag 22) mit Minocyclin, Pentaacetylminocyclin oder Vehikel (Kontrolle) behandelt wurden (je 100 µg/Tag). Dargestellt sind Mittelwerte und SEM von jeweils 5 Mäusen.
Figur 8 zeigt die Anzahl entzündlicher Immunzellinfiltrate im Rückenmark von Minocyclin und Pentaacetylcyclin i.p. und oral behandelter Mäuse (n = 4; Applikation von Tag 13 bis Tag 17, Analyse der Rückenmarksinfiltrate am Tag 20 nach Induktion der EAE). Dargestellt sind die Mittelwerte und Standardfehler der Mittelwerte.

Literaturliste:
1. Yong et al. (2004), Lancet Neurol. 3, 744-751.
2. Nelissen et al. (2003), Brain. 126, 1371-1381.
3. Morimoto et al. (2005), Brain Res. 1044, 8-15. Epub 2005 Apr 2013.
4. Kraus et al. (2005), J Neurochem. 94, 819-827.
5. Jordan et al. (2007), Curr Drug Deliv 4, 225-231.
6. Diguet et al. (2004), Exp Neurol. 189, 1-4.
7. Mansson et al. (2010), Hepatology 51, 347-348; author reply 349-350.
8. Ramakrishna, (2009), J Clin Gastroenterol 43, 787-790.
9. Gough et al. (1996), BMJ 312, 169-172.
10. Bhat et al. (1998), J Clin Gastroenterol 27, 74-75.
11. Chang et al. (2010), J Autoimmun 34, J266-275.

### Beispiele

Ein A-Ring-aromatisiertes Minocyclin entsprechend der Formel I wurde nach Beispiel 1 der WO 2009/01741 A1 hergestellt. Zur weiteren Untersuchung der Wirkung dieser Substanz erfolgte eine Aufreinigung (Reinheit 95%) entsprechend dem Stand der Technik. Alle nachfolgend aufgeführten Beispiele wurden mit dem fünffach acetylierten A-Ring-aromatisierten Minocyclin, also Pentaacetylcyclin, der Formel II durchgeführt.

### Beispiel 1: in vitro-Untersuchungen zum Einfluss von Pentaacetylcyclin auf die Proliferation (Zellteilung) stimulierter humaner T-Lymphozyten

In *in* vitro-Untersuchungen dienten humane T-Lymphozyten des peripheren Blutes gesunder Spender zur Testung der immunmodulatorischen/immunsuppressiven Wirkung der Testsubstanzen. Eine gesteigerte Zellteilung/Proliferation dieser Immunzellen nach Stimulation simuliert *in vitro* eine Entzündungsreaktion. Eine Verringerung dieser stimulierten Proliferation durch applizierte Substanzen veranschaulicht deren immunmodulatorischen/immunsuppressiven Eigenschaften.

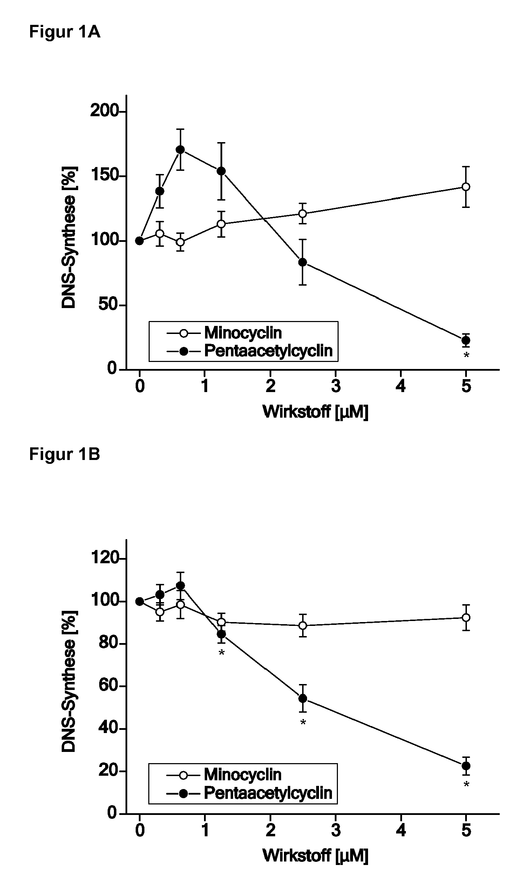

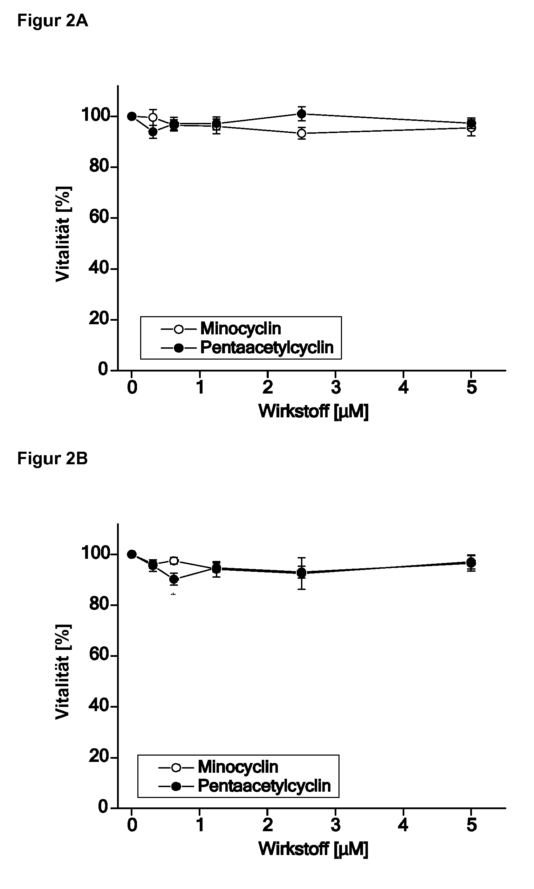

Humane T-Lymphozyten wurden in serumfreiem Kulturmedium mit anti-CD3-Antikörpern (Klon OKT3) bzw. mit Pokeweed-Mitogen (PWM, 2 µg/ml) in An- und Abwesenheit verschiedener Konzentrationen von Minocyclin oder Pentaacetylcyclin stimuliert. Die Ermittlung der Proliferation (DNS-Synthese) der Zellen erfolgte nach 72 Stunden über den Einbau von 3H-Thymidin. Zur Überprüfung der Vitalität der Zellkulturen wurde der CeIITiter-Blue® Cell Viability-Assay (Promega) verwendet. Diese *in vitro*-Untersuchungen an humanen T-Lymphozyten belegen, dass Minocyclin in dem getesteten Konzentrationsbereich keinen signifikanten Einfluss auf die Proliferation (DNS-Synthese) ausübt. Im Gegensatz dazu zeigte Pentaacetylcyclin in einem wirksamen Konzentrationsbereich von 2,5 bis 5,0 µM eine signifikante Hemmung der Proliferation der humanen T-Lymphozyten nach Stimulation mit PWM (Figur 1A) oder anti-CD3-Antikörpern (Figur 1 B). Diese bis auf ca. 20% reduzierte Zellteilung deutet auf eine starke immunsuppressive Wirkung von Pentaacetylcyclin hin. Die Vitalität der T-Lymphozyten in diesen Zellsystemen war bis zu Konzentrationen von 5,0 µM Minocyclin bzw. Pentaacetylcyclin nicht beeinträchtigt (PWM-Stimulation, Figur 2A; anti-CD3-Stimulation, Figur 2B).

### Beispiel 2: In vitro-Untersuchungen zum Einfluss von Pentaacetylcyclin auf die Zytokinproduktion stimulierter humaner T-Lymphozyten

Zytokine stellen Indikatoren für die Stärke einer Entzündungsreaktion dar und sind aktive Regulatoren der Differenzierung und Proliferation von T-Lymphozyten. Aus diesem Grund lassen sich aus den in einem *in* vitro-Testsystem gewonnenen Daten zur Produktion/Freisetzung von Zytokinen detailliertere Aussagen über die immunmodulierenden/immunsuppressiven Eigenschaften von Testsubstanzen herleiten.

Humane T-Lymphozyten des peripheren Blutes gesunder Spender wurden in serumfreiem Kulturmedium mit Pokeweed-Mitogen (PWM, 2 µg/ml) stimuliert und in An- und Abwesenheit verschiedener Konzentrationen von Minocyclin oder Pentaacetylcyclin für 48 Stunden inkubiert. Anschließend erfolgte die Messung der Konzentrationen von Interferon-y (IFN-y), Interleukin-2 (IL-2) und Interleukin-17 (IL-17) in den Zellkulturüberständen mittels spezifischer ELISA (R&D Systems).

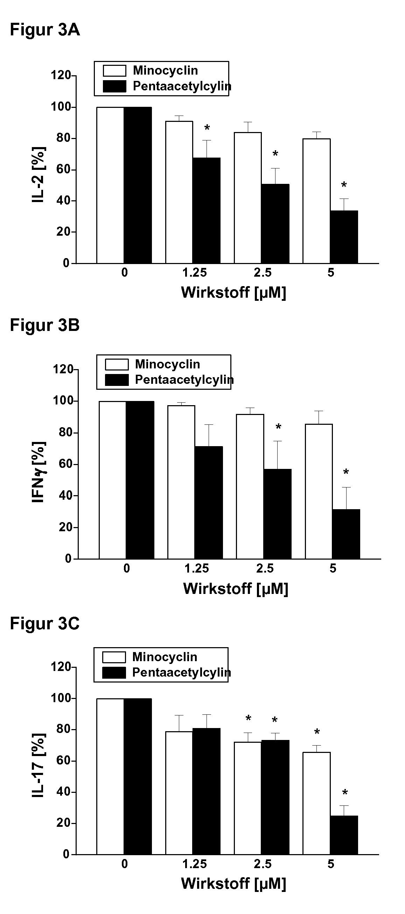

Diese *in vitro*-Untersuchungen erbrachten den Befund, dass Minocyclin keinen signifikanten Einfluss auf die Produktion und Sekretion der genannten T-Zell-Zytokine dieser Zellen ausübt. Überraschenderweise konnte jedoch nach Inkubation mit 2,5 bis 5,0 µM Pentaacetylminocyclin eine signifikante Hemmung der Produktion/Freisetzung der Zytokine IL-2 (Figur 3A), IFN-γ (Figur 3B) und IL-17 (Figur 3C) beobachtet werden.

### Beispiel 3: In vitro-Untersuchungen zum Einfluss von Pentaacetylcyclin auf die Proliferation (Zellteilung) von Maus-Splenozyten

In weiteren in vitro-Untersuchungen dienten Splenozyten (Milzzellen) von SJL/J-Mäusen zur Testung der immunmodulatorischen/immunsuppressiven Wirkung der Testsubstanzen. Nach Stimulation der Splenozyten mit Pokeweed-Mitogen (PWM, 1 µg/ml) oder mit anti-CD3-Antikörpern (Klon 145-2C11, BD Biosciences) erfolgte die Inkubation dieser Immunzellen in An- oder Abwesenheit verschiedener Konzentrationen von Minocyclin und Pentaacetylcyclin. Die anschließende Proliferation (DNS-Synthese) der Zellen wurde nach 72 Stunden über den Einbau von radioaktivem 3H-Thymidin ermittelt. Gleichzeitig erfolgte die Überprüfung der Vitalität der Zellkulturen mittels des CeIITiter-Blue® Cell Viability-Assays (Promega).

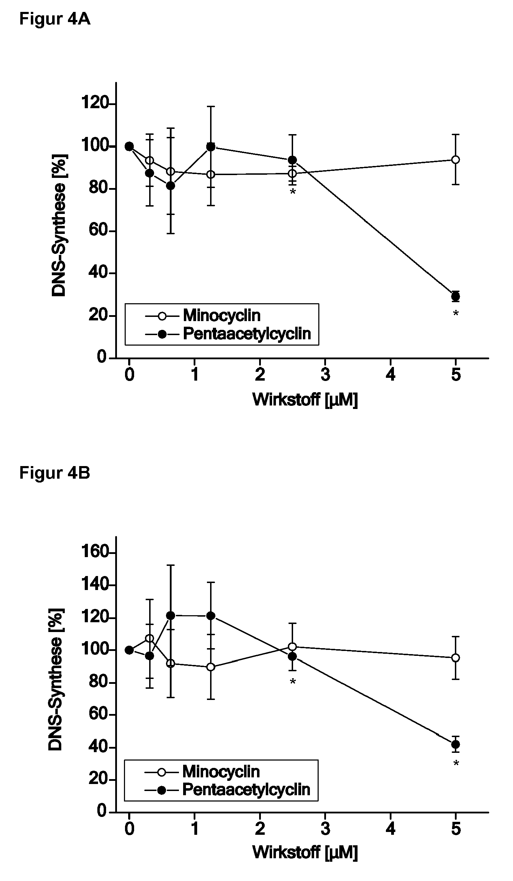

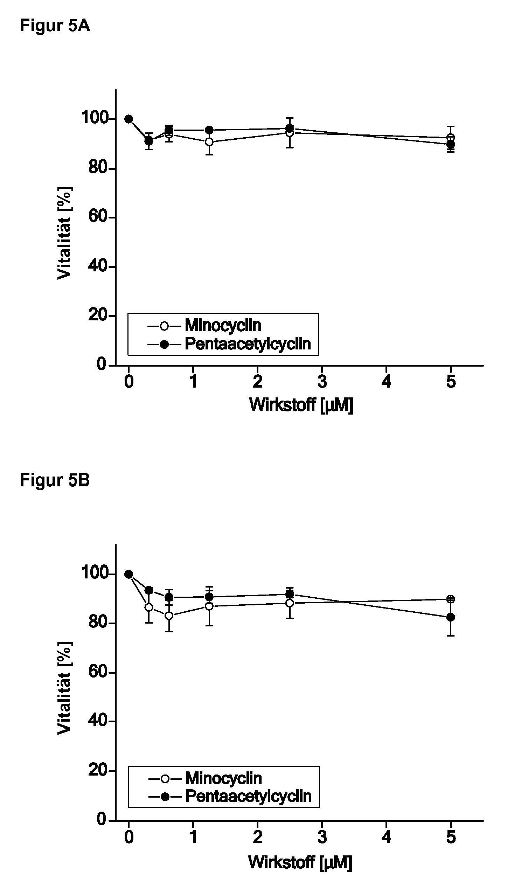

Übereinstimmend mit den Ergebnissen der Untersuchungen an den humanen T-Lymphozyten konnte auch an stimulierten Splenozyten der Maus eine signifikante Hemmung der Proliferation, erkennbar an der verminderten DNS-Synthese, nach Applikation von 2,5 bzw. 5,0 µM Pentaacetylcyclin nachgewiesen werden (Figur 4). Die bis auf ca. 30% reduzierte Zellteilung bestätigt die bereits an humanen T-Zellen erhaltenen Ergebnisse zur immunsuppressiven Wirkung von Pentaacetylcyclin. Im Gegensatz dazu zeigte Minocyclin in diesem Konzentrationsbereich, unabhängig vom verwendeten Stimulus, keinerlei Wirkung (PWM-Stimulation, Figur 4A; anti-CD3-Stimulation, Figur 4B). Die unter gleichen Bedingungen getestete Vitalität der Splenozyten zeigte bis 5,0 µM Pentaacetylcyclin keine Veränderung (PWM-Stimulation, Figur 5A; anti-CD3-Stimulation, Figur 5B).

### Beispiel 4: In vitro-Untersuchungen zum Einfluss von Pentaacetylcyclin auf die Zytokinproduktion von Maus-Splenozyten

Zur Verifizierung der an humanen Zellen erhaltenen Ergebnisse hinsichtlich der reduzierten Zytokinproduktion erfolgten ebenfalls *in* vitro-Untersuchungen an Splenozyten (Milzzellen) der SJL/J-Mäuse. Diese Splenozyten von wurden mit Pokeweed-Mitogen (PWM, 1 µg/ml) stimuliert und in An- oder Abwesenheit verschiedener Konzentrationen von Minocyclin und Pentaacetylcyclin inkubiert. Nach 48 Stunden erfolgte die Analyse der Zellkulturüberstände hinsichtlich der Freisetzung der Zytokine Interferon-y (IFN-y) und Interleukin-17 (IL-17) mittels spezifischer ELISA (R&D Systems).

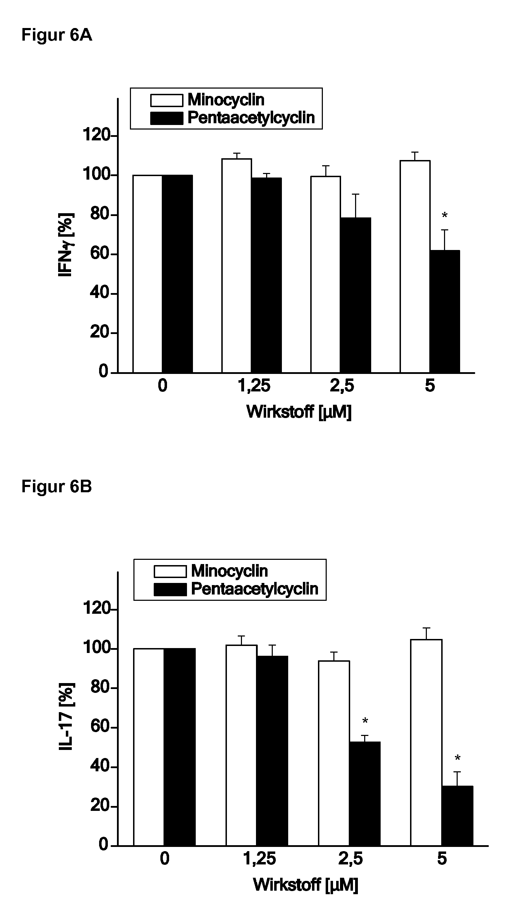

Es konnte auch in diesem Zellsystem gezeigt werden, dass Minocyclin im getesteten Konzentrationsbereich keinen Einfluss auf die Zytokinproduktion (IFN-γ, IL-17) von Maus-Splenozyten hat. Hingegen war eine signifikante Hemmung der genannten Parameter nach Inkubation mit Pentaacetylcyclin in einem wirksamen Konzentrationsbereich von 2,5 bis 5,0 µM zu beobachten (Figur 6). Die drastische Reduktion der Freisetzung von IFN-γ (auf ca. 60%, Figur 6A) und von IL-17 (auf 20 - 30%, Figur 6B) nach Applikation von 5,0 µM Pentaacetylminocyclin ist ein eindeutiger Hinweis auf die immunsuppressive Wirkung der erfindungsgemäßen Wirksubstanz.

### Beispiel 5: In vivo-Untersuchungen im Mausmodell für die humane Multiple Sklerose (MS), der Experimentellen Autoimmunen Enzephalomyelitis (EAE)

Aufgrund der immunpathologischen Ähnlichkeiten der MS mit der EAE von Nagetieren stellt das etablierte in vivo-Mausmodell der EAE ein akzeptiertes Tiermodell für die humane MS dar. Zudem gilt die EAE als Prototyp für T-Lymphozyten-vermittelte, organspezifische Autoimmunerkrankungen. Insbesondere IFN-γ/IL-2-produzierende (sogenannte Th1-Zellen) und IL-17-produzierende (sogenannte Th17-Zeiien) T-Lymphozyten nehmen eine Schlüsselstellung bei der Entstehung der EAE und der MS ein.

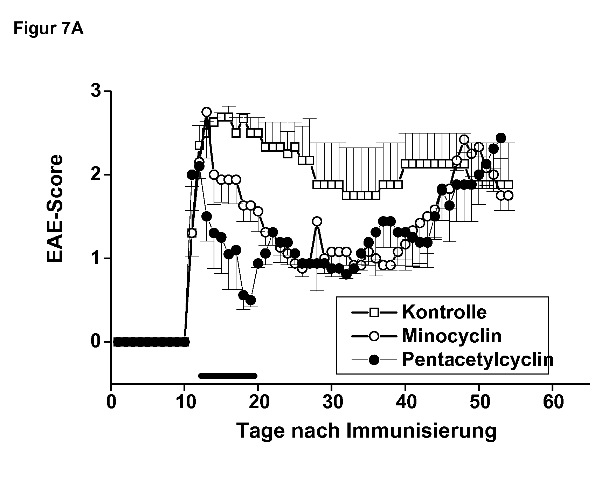

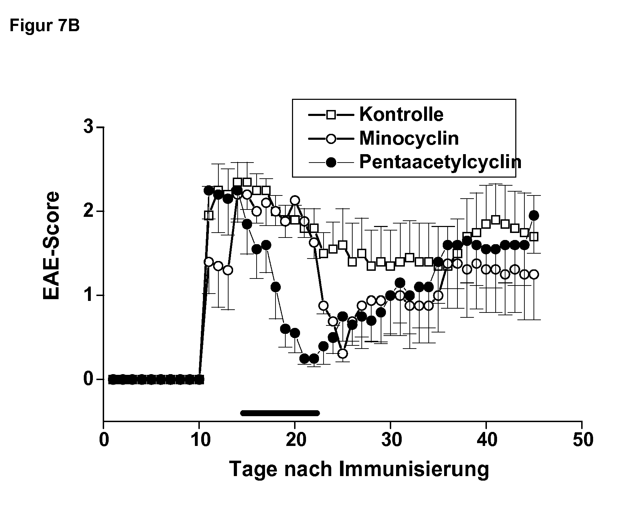

Die EAE wurde in einem suszeptiblen Tierstamm (SJL/J-Maus) nach Immunisierung mit einem Myelinprotein-Peptid (Proteolipid-Protein 139-151, PLP 139-151) induziert und die Applikation von Minocyclin, Pentaacetylcyclin und einem Kontroll-Vehikel erfolgte in einem therapeutischen Ansatz (nach eindeutiger Ausbildung der EAE-Erkrankung) in zwei getrennten Versuchsansätzen:
1.) intraperitoneal (i.p., 100 µg/Tag) von Tag 12 bis Tag 20 (Figur 7A)
2.) oral (100 µg/Tag) von Tag 14 bis Tag 22 (Figur 7B).

Als Maß für die Wirkung der therapeutischen Applikation wurde täglich der Schweregrad der EAE-Erkrankung (EAE-Score) bestimmt.

Sowohl Minocyclin als auch Pentaacetylcyclin hatten bei intraperitonealer Verabreichung einen signifikanten therapeutischen Effekt auf die akute Phase der EAE in SJL/J-Mäusen. Der therapeutische Effekt auf die akute Phase der EAE trat jedoch nach i.p.-Applikation von Pentaacetylcyclin etwa 5 bis 6 Tage früher auf als bei Minocyclin (Figur 7A).

Interessanterweise hatten beide Substanzen auch bei therapeutischer oraler Applikation einen signifikanten Einfluss auf die akute Phase der EAE. Überraschend ist, dass der positive Effekt von Pentaacetylcyclin auf die akute Phase der EAE etwa 4 bis 5 Tage früher zu beobachten war als bei Minocyclin (Figur 7B).

Diese eindeutig bessere und schnellere Schutzwirkung von Pentaacetylcyclin im genutzten in vivo-Testsystem der MS ist ein völlig neuartiger Befund und unterstreicht die immunmodulatorischen/immunsuppressiven Effekte der Substanz aus den zuvor beschriebenen in vitro-Experimenten.

### Beispiel 6: Anzahl entzündlicher Infiltrate im Rückenmark von SJL/J-Mäusen nach Experimenteller Autoimmuner Enzephalomyelitis (EAE)

Als Folge einer T-Lymphozyten-Aktivierung und -Differenzierung kommt es bei der EAE zu einer massiven Einwanderung (Infiltration) von Immunzellen in das Rückenmark der Mäuse. Diese Immunzellen vermitteln durch Demyeliniserung und neuronale Schädigung die weitere Erkrankung und lassen sich auf histologischen Schnitten als entzündliche Infiltrate darstellen und analysieren. Die Stärke der Infiltration von Immunzellen in das Rückenmark während der EAE korreliert im Allgemeinen mit dem Schweregrad der EAE-Erkrankung.

Deshalb erfolgte die histologische Analyse der entzündlichen Infiltrate im Rückenmark von SJL/J-Mäusen nach EAE und 5-tägiger therapeutischer Applikation von Minocycline bzw. Pentaacetylcyclin.

Die EAE wurde in SJL/J-Mäusen durch Immunisierung mit einem Myelinprotein-Peptid (Proteolipid-Protein 139-151, PLP 139-151) induziert. Die Applikation von Minocyclin, Pentaacetylcyclin und einem Kontroll-Vehikel erfolgte in einem therapeutischen Ansatz (nach eindeutiger Ausbildung der EAE-Erkrankung) in zwei getrennten Versuchsansätzen:
1.) intraperitoneal (i.p., 100 µg/Tag) von Tag 13 bis Tag 17
2.) oral (100 µg/Tag) von Tag 13 bis Tag 17.

Am Tag 20 nach Immunisierung erfolgte die Aufarbeitung des Rückenmarks der Mäuse für histologische Untersuchungen (Paraffin-Schnitte, HE-Färbungen). Als Maß für die Wirkung der therapeutischen Applikation wurde die Anzahl entzündlicher Immunzellinfiltrate (Läsionen) im Rückenmark therapierter und nicht therapierter Mäuse verglichen.

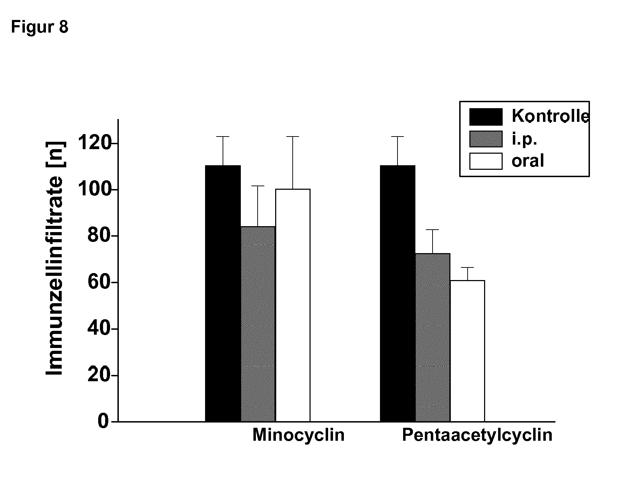

Die Auswertungen der histologischen Präparate des Rückenmarks von behandelten und unbehandelten Mäusen zeigten eine Übereinstimmung des klinischen Befundes (Schweregrad der EAE, Figur 7) mit der Rückenmarkshistologie (Anzahl von Immunzellinfiltraten, Figur 8). Es konnte nach einer nur fünftägigen intraperitonealen bzw. oralen Therapie mit beiden Substanzen aber insbesondere mit Pentaacetylcyclin eine deutliche Verringerung der Zahl der entzündlichen Infiltrate im Rückenmark beobachtet werden.

## Patentansprüche

1. Ein A-Ring-aromatisiertes Acetylminocyclin der Formel I 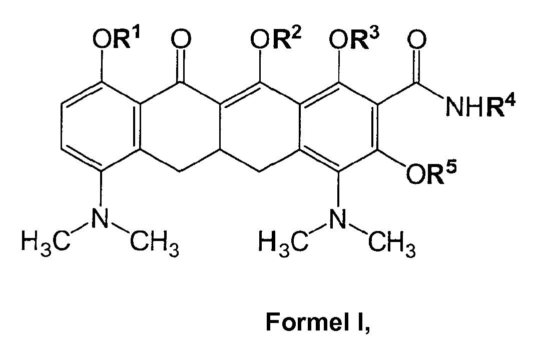 wobei R¹ bis R⁵ unabhängig voneinander = Acetyl und/oder H und wenigstens ein Rest R von R¹ bis R⁵ Acetyl ist, zur Verwendung in einem therapeutischen Verfahren zur Therapie von T-Zell-vermittelten Krankheiten.

2. A-Ring-aromatisiertes Acetylminocyclin gemäß Anspruch 1, wobei die T-Zell-vermittelte Krankheit eine T-Zell-vermittelte Allergie ist.

3. A-Ring-aromatisiertes Acetylminocyclin gemäß Anspruch 1, wobei die T-Zell-vermittelte Krankheit eine Autoimmunerkrankung ist.

4. A-Ring-aromatisiertes Acetylminocyclin gemäß Anspruch 1, wobei die T-Zell-vermittelte Krankheit eine bei einer Transplantation auftretende Abstoßungsreaktion ist.

5. Ein A-Ring-aromatisiertes Acetylminocyclin der Formel I 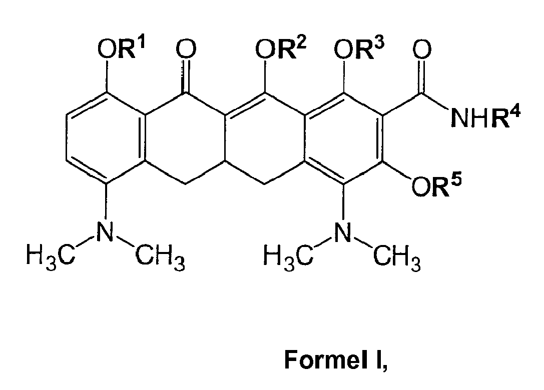 wobei R¹ bis R⁵ unabhängig voneinander Acetyl und/oder H und wenigstens ein Rest R von R¹ bis R⁵ Acetyl ist, zur Verwendung in einem therapeutischen Verfahren zur Suppression der Immunreaktion eines Patienten.

6. A-Ring-aromatisiertes Acetylminocyclin gemäß Anspruch 5, wobei die Suppression der Immunreaktion eine Reduktion der T-Lymphozytenproliferation und/oder eine Reduktion der T-Zell-Zytokin-Bildung ist.

7. Das A-Ring-aromatisierte Acetylminocyclin gemäß der Ansprüche 1 bis 6, wobei dieses Pentaacetylcyclin oder Tetraacetylcyclin ist.

8. Eine pharmazeutische Zusammensetzung zur Verwendung in einem therapeutischen Verfahren zur Therapie von T-Zell-vermittelten Krankheiten, umfassend wenigstens ein A-Ring-aromatisiertes Acetylminocyclin der Formel I 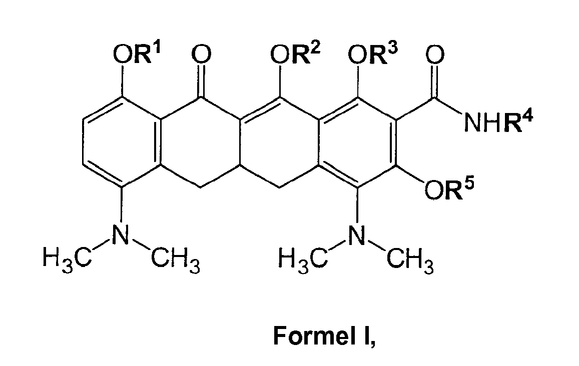 wobei R¹ bis R⁵ unabhängig voneinander Acetyl oder H und wobei wenigstens ein Rest R von R¹ bis R⁵ Acetyl ist, und einen pharmazeutisch verträglichen Träger.

9. Eine pharmazeutische Zusammensetzung zur Verwendung in einem therapeutischen Verfahren zur Suppression der Immunreaktion in einem Patienten, umfassend wenigstens ein A-Ring-aromatisiertes Acetylminocyclin der Formel I 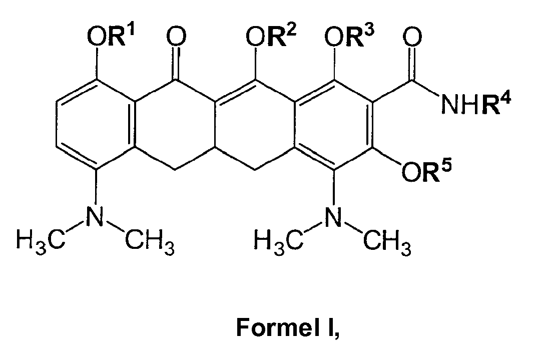 wobei R¹ bis R⁵ unabhängig voneinander Acetyl oder H und wenigstens ein Rest R von R¹ bis R⁵ Acetyl ist, und einen pharmazeutisch verträglichen Träger.
